# EUROPEAN PATENT SPECIFICATION

(11) **EP 2 152 139 B1**
(45) Date of publication and mention of the grant of the patent: **01.11.2017**
(21) Application number: 08769739.7
(22) Date of filing: 27.05.2008
(51) Int. Cl.: A61B 1/005, A61M 25/01

(54) **ACTIVE CONTROLLED BENDING IN MEDICAL DEVICES**
AKTIV GESTEUERTE BIEGUNG BEI MEDIZINISCHEN VORRICHTUNGEN
FLEXION ACTIVE CONTRÔLÉE DANS DES DISPOSITIFS MÉDICAUX

(30) Priority: 31.05.2007 US 932413 P
(43) Date of publication of application: 17.02.2010
(73) Proprietor: Boston Scientific Limited, Hamilton HM11 (BM)
(72) Inventor: INTOCCIA, Alfred P., Nashua, New Hampshire 03062 (US); MCINTYRE, Jon T., Newton, Massachusetts 02465 (US); SLANDA, Jozef, Milford, Massachusetts 01757 (US)
(74) Representative: Pfenning, Meinig & Partner mbB
(86) International application number: PCT/US2008/064867
(87) International publication number: WO 2008/150767

(56) References cited:
- EP-A- 0 422 887
- WO-A-94/10897
- WO-A-2005/094661
- WO-A-2006/034008
- DE-A1- 10 052 679
- FR-A- 2 713 492
- JP-A- 61 118 712
- US-A- 5 381 782
- US-A- 6 012 494
- US-A1- 2006 041 188

## Description

### TECHNICAL FIELD

The present invention generally relates to medical devices such as endoscopes and catheters. More specifically, the invention relates to flexible medical devices that are bendable and steerable in order to negotiate and access various areas within a patient.

### BACKGROUND INFORMATION

It has become well established that there are major public health benefits from early detection and treatment of disease of internal organs such as the alimentary and excretory canals and airways, e.g., the colon, esophagus, stomach, urethra, bladder, ureter, kidney, lungs, bronchi, uterus, and other organ systems. Early detection of such diseases can be accomplished by periodic medical examinations aided by modem medical procedures and devices such as an endoscope. A conventional imaging endoscope used for such procedures generally comprises a flexible tube with a fiber optic light guide that directs illuminating light from an external light source to the distal tip where it illuminates the region (i.e., tissue, occlusive objects) to be examined. Frequently, additional optical components are incorporated to adjust the spread of the light exiting the fiber bundle and the distal tip. An objective lens and fiber optic imaging light guide communicating with a camera at the proximal end of the endoscope, or an imaging camera chip at the distal tip, produce an image that is displayed to the operator. In addition, most endoscopes include one or more working channels through which medical devices such as biopsy forceps, snares, fulguration probes, and other tools may be passed.

Some endoscopes and electrophysiology catheters have means for steering or deflecting the distal tip of the endoscope to follow the pathway of the anatomy under examination such as the colon, bladder, kidney, and heart. Deflection or articulation is often a desirable characteristic in these types of medical devices to minimize friction force and trauma to the surrounding tissue, and to survey targeted examination sites. Navigation of the endoscope through various areas within a patient improves the success of the examination and minimizes pain, side effects, risk, or sedation to the patient.

In order to achieve active deflection at the distal flexible portion of the endoscope, control cables or wires are carried within the endoscope shaft connecting the distal end to a set of controls in a handle. By manipulating the controls, the operator is able to steer the endoscope during insertion and direct it to a region of interest.

For example, publication DE 100 52 679 A1 discloses an endoscope comprising a control section from which a shaft extends. The shaft has a frame with a one-piece pipe along the main part of the shaft's length. The pipe consists of a super-elastic alloy and has slits at least over a section.

There are many design and performance challenges inherent in these devices. Some of these challenges include achieving planar deflection at the tip as well as preventing the shaft from buckling or forming a series of "S" shapes from the tension of pull wire mechanisms. Other challenges faced by the designers of these devices include being able to keep an individual bend in one plane, achieving the appropriate amount of angular deflection and achieving multiple directions of deflection.

Typically, flexible endoscopes are very expensive medical devices. Because of the expense, these endoscopes are built to withstand multiple uses upon many patients and repeated disinfections. Conventional endoscopes are generally built of strong composite material structures such as metals and plastics that do not degrade under repeated cleaning and high temperatures. These material structures decrease the flexibility of the endoscope and can compromise patient comfort. Furthermore, conventional endoscopes are complex and fragile instruments that frequently need expensive repair as a result of damage during use or during a disinfection procedure.

To overcome these and other problems, the development of a low cost endoscope would allow endoscopes to be used for a single procedure and then disposed, eliminating the need for preparation and cleaning and increasing the total volume of endoscopes required. This larger volume would enable the manufacturer to achieve economies of scale and to incorporate manufacturing methods that are not economical when
used in current volumes and are only economical in large volumes (100,000 units/per year). The low cost endoscope should be packaged sterile or disinfected and be capable of being used for a single procedure without endoscope preparation and then discarded. The endoscope should include one or more of the following features: better navigation and tracking, a superior interface with the operator, improved access by reduced frictional forces upon the lumenal tissue, increased patient comfort, greater clinical productivity and patient throughput than is currently available with a conventional endoscope, a lower risk of cross-contamination and the ability to be used across more procedures.

### SUMMARY OF THE INVENTION

It thus would be desirable to provide a new device with active controlled bending and methods for making flexible shafts for medical devices. It would be particularly desirable to provide such a device and method that would achieve planar deflection at the tip as well as preventing the shaft (non-deflecting portion) from buckling or forming a series of "S" shapes from the tension of pull wire mechanisms in comparison to prior art devices. It also would be desirable to provide such a device that would be able to keep an individual bend in one plane, achieve the appropriate amount of angular deflection and achieve multiple directions of deflection. Such deflection devices would be simple in construction and less costly than prior art devices, and such methods would not require highly skilled users to utilize the device.

A particular embodiment of the present invention relates to a flexible endoscope having a handle and a flexible shaft extending from the handle. The shaft includes a distal portion having a tubular wall defining a central lumen and a least two smaller lumens extending longitudinally through at least a portion of the tubular wall and a pull wire is disposed within each of the smaller lumens. The distal portion further includes an articulation layer disposed over the tubular wall and includes a first series of slots, which allow controlled bending of the distal portion by movement of one or more of the pull wires.

In an alternative embodiment of the present invention, the distal portion further includes a second series of slots. The second series of slots may be offset from the first series of slots, which allows controlled bending of the distal portion in more than one plane. The spacing between the slots in the first series of slots may be the same or different from the spacing between the slots in the second series of slots. Similarly, the slot width of the first series of slots may be the same or different from the slot width of the second series of slots. By varying the spacing between the slots and/or the slot width the bending characteristics in different planes can be customized. In addition, the geometric shape of the slots (e.g., rounded or squared) can be varied to further customize the bending characteristics of the distal portion.

In another aspect of the invention, the endoscope of the present invention further includes an outer sleeve disposed on the outside of the flexible shaft to provide a smooth exterior surface. A variety of lubrications and/or drug coatings can also be included on the outer sleeve to reduce friction or treat portions of the patient being examined.

In a further aspect of the invention, the handle of the endoscope further includes a control system. The control system may include, for example, knobs, hubs, or levers attached to the pull wires to assist in controlled bending of the distal portion by movement of the control system.

In yet another aspect of the invention, the endoscope of the present invention further includes radiopaque markers or radiopaque materials when fluoroscopy is being utilized to ensure proper positioning of the endoscope.

### BRIEF DESCRIPTION OF THE DRAWINGS

For a fuller understanding of the nature and operation of various embodiments according to the present invention, reference is made to the following description taken in conjunction with the accompanying drawing figures wherein like reference characters denote corresponding parts throughout the several views and wherein:
FIG. 1 depicts a schematic rendering of an endoscope incorporating features of the present invention;
FIG. 2A depicts a schematic rendering of the distal portion of the endoscope shown in FIG. 1 with the first active deflection section in a straight position and the second active deflection section bent downward;
FIG. 2B depicts a schematic rendering of the distal portion of the endoscope shown in FIG. 1 with the first active deflection section bent downward and the second active deflection section in a straight position;
FIG. 2C depicts a schematic rendering of the distal portion of the endoscope shown in FIG. 1 with the first active deflection section bent to the left and the second active deflection section bent downward;
FIG. 2D depicts a schematic rendering of the distal portion of the endoscope shown in FIG. 1 with the first active deflection section bent to the right and the second active deflection section bent upward;
FIG. 2E depicts a schematic rendering of the distal portion of the endoscope shown in FIG. 1 with the first active deflection section bent to the right and the second active deflection section bent downward;
FIG. 2F depicts a schematic rendering of the distal portion of the endoscope shown in FIG. 1 with the first active deflection section bent to the left and the second active deflection section bent upward;
FIG. 3 depicts an exploded rendering of the handle of the endoscope shown in FIG. 1;
FIG. 4 depicts an enlarged schematic rendering of the assembled right side articulation hub shown in FIG. 3;
FIG. 5 depicts a perspective view of the distal portion of the flexible shaft partially cut-away exposing the first and second active deflection sections according to one embodiment of the present invention;
FIG. 6 depicts a partial cut-away side view of the first active deflection section of FIG. 5 shown from the perspective indicated by line 6;
FIG. 7 depicts a partial cut-away top view of the second active deflection section of FIG. 5 shown from the perspective indicated by line 7;
FIG. 8 depicts a cross section of the distal portion of the flexible shaft of FIG. 5 taken along the section line 8-8;
FIG. 9 depicts a cross section of the distal portion of the flexible shaft of FIG. 5 taken along the section line 9-9;
FIG. 10 depicts a schematic rendering of the distal portion of the flexible shaft of FIG. 1 with the second active deflection section bent in a downward direction;
FIG. 11 is depicts a schematic rendering of the distal portion of the flexible shaft of FIG. 1 with the first active deflection section bent downward and the second active deflection section in a straight position;
FIG. 12 depicts a side view of the distal portion of the flexible shaft partially cut-away exposing the first and second active deflection sections;
FIG. 13 depicts a cross section of the distal portion of the flexible shaft of FIG. 12 taken along the section line 13-13;
FIG. 14 depicts a cross section of the distal portion of the flexible shaft of FIG. 12 taken along the section line 14-14;
FIG. 15 depicts a cross section of the distal portion of the flexible shaft of FIG. 12 taken along the section line 15-15;
FIG. 16 depicts a schematic rendering of the distal portion of the flexible shaft of FIG. 12;
FIG. 17 depicts a schematic rendering of the distal portion of the flexible shaft of FIG. 12;
FIG. 18 depicts a side view of the distal portion of the flexible shaft partially cut-away exposing the first and second active deflection sections;
FIG. 19 depicts a cross section of the distal portion of the flexible shaft of FIG. 18 taken along the section line 19-19;
FIG. 20 depicts a cross section of the distal portion of the flexible shaft of FIG. 18 taken along the section line 20-20;
FIG. 21 depicts a cross section of the distal portion of the flexible shaft of FIG. 18 taken along the section line 21-21;
FIG. 22 depicts a schematic rendering of the distal portion of the flexible shaft of FIG. 18; and
FIG. 23 depicts a schematic rendering of the distal portion of the flexible shaft of FIG. 18.

### DESCRIPTION

As indicated above, the present invention is a flexible endoscope that allows an operator to access, and view internal body anatomy of a patient as well as to insert surgical instruments into the patient's body. In addition, the endoscope may include integrated diagnostic and therapeutic capabilities to allow the operator to treat the patient in a single procedure. An endoscope of the present invention can be sufficiently inexpensive to manufacture such that the endoscope can be considered a single use, disposable item.

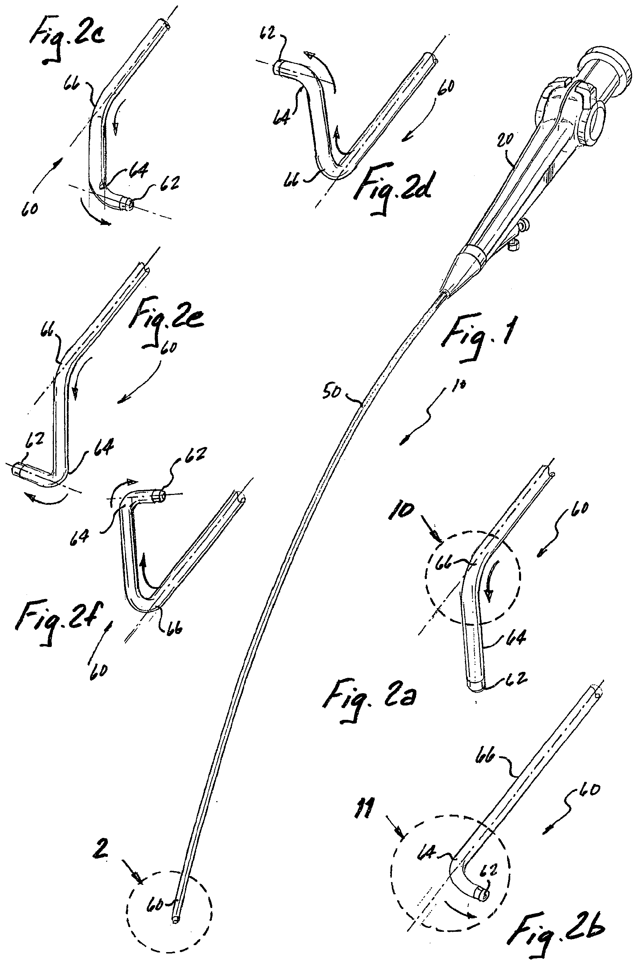

Referring now to FIG. 1, an endoscope 10 according to one embodiment of the present invention includes a handle 20 at the proximal end of the endoscope 10 and a flexible shaft 50 extending distally from the handle 20. The terms proximal and distal require a point of reference. In this application, the point of reference is the perspective of the user. Therefore, the term proximal will always refer to an area closest to the user, whereas distal will always refer to an area away from the user.

Referring now also to FIGS. 2A-2F, the flexible shaft 50 has a distal portion 60 with predictable and planar active deflection capability. This deflection can be achieved in multiple directions and at multiple points along the axial orientation. As shown, the distal portion 60 is capable of active deflection in two distinct planes. FIGS. 2C-2F show bending in a first plane of deflection relative to the handle 20 (up and down) and a second plane of deflection (right and left) that is substantially orthogonal to the first plane of deflection. All relative descriptions herein such as top, bottom, left, right, up, and down are with reference to the figures, and thus should not be construed in a limiting sense.

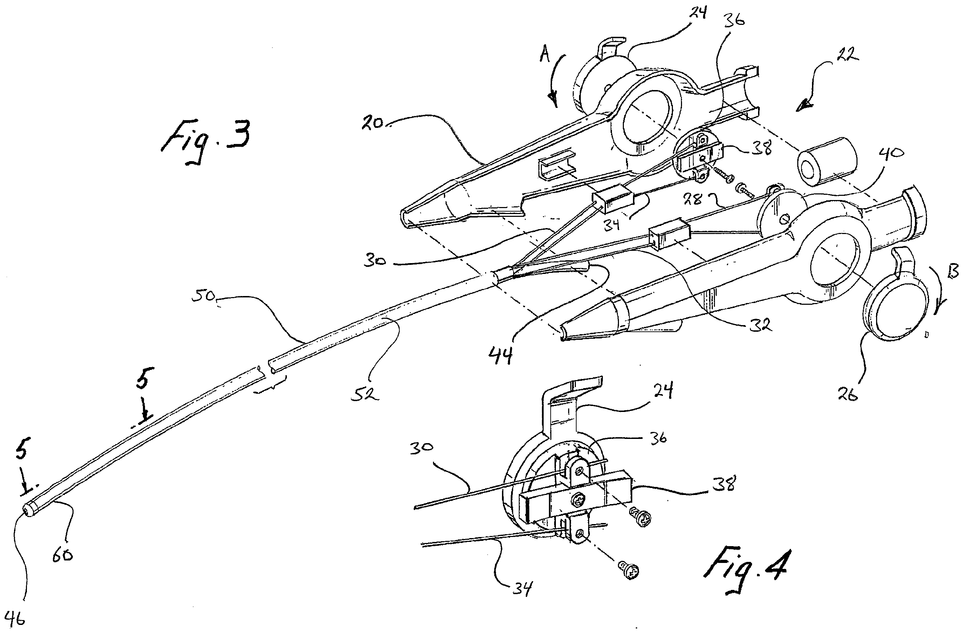

Referring now to FIGS. 3 and 4, the handle 20 includes a control system 22 to control the active deflection capability of the distal portion 60 of the flexible shaft 50. The control system 22 comprises two activation hubs 24, 26, and four pull wires 28, 30, 32, 34. Each activation hub 24, 26 is connected to two of the pull wires and allows the user to manipulate the distal portion 60 of the flexible shaft 50 in one plane of deflection. Additional activation hubs and/or pull wires could be included in the control system 22 depending on how many planes of deflection are desired. The pull wires 28, 30, 32, 34 are made from stainless steel, polymer filaments, or other metals and alloys such as, for example, *Nitinol.*

The first activation hub 24 is movably attached to the right side of the handle 20 from the perspective of the user and includes a floating cam 36 and a cam stop 38. The proximal ends of pull wires 30 and 34 are connected to the floating cam 36. When the user rotates the first activation hub 24 in a clockwise direction as indicated by line A on FIG. 3, tension is applied to pull wire 34, and tension is released from pull wire 30, thereby deflecting the distal portion 60 of the flexible shaft 50 to the left. Conversely, when the user rotates the first activation hub 24 in the opposite, counter-clockwise direction, tension is applied to pull wire 30 and tension is released from pull wire 34, thereby deflecting the distal portion 60 to the right.

The user can achieve up and down deflection of the distal portion 60 of the flexible shaft 50 by rotating the second activation hub 26 in a similar manner. The second activation hub 26 is movably attached to the left side of the handle 20 from the perspective of the user and includes a floating cam 40 and a cam stop (not shown). The proximal ends of pull wires 28 and 32 are connected to floating cam 40. When the user rotates the second activation hub 26 in a clockwise direction as indicated by line B on FIG. 3, tension is applied to pull wire 28, and tension is released from pull wire 32, thereby deflecting the distal portion 60 in an upward direction. Conversely, when the user rotates the second activation hub 26 in the opposite, counter-clockwise direction, tension is applied to pull wire 32 and tension is released from pull wire 28, thereby deflecting the distal portion 60 in a downward direction. The control system 22 could comprise additional components or alternative means for achieving defection of the distal portion 60 of the flexible shaft 50.

The handle 20 also includes a working port hub 44. The working port hub 44 provides access to the working channel 46 of the endoscope 10. The working channel 46 extends from the working port hub 44 to the distal end 62 of the flexible shaft 50 and is used to insert ancillary products such as, for example, guide wires, graspers, cutters, irrigation, laser fibers and the like to facilitate a variety of diagnostic and therapeutic procedures. In alternative embodiments, the working channel 46 may comprise one single central lumen or may be further subdivided into a plurality of smaller lumens of various shapes and sizes to accommodate different ancillary products.

The portion of the flexible shaft 50 proximal to the distal portion 60 may comprise any suitable type of flexible shaft, such as the shaft disclosed in U.S. patent application Ser. No. 10/956,011 (U.S. Patent Publication No. 2005-0131279). The flexible shaft 50 may be uniformly flexible or could comprise a plurality of segments having varying degrees of flexibility or rigidity. The flexible shaft 50 includes an outer sleeve 52 disposed on the outside of the flexible shaft 50 to provide a smooth exterior surface. The outer sleeve 52 can be made from soft, thin polyurethane, LLDPE, silicon, pellethane, polyurethane or other approved biocompatible materials such as polyethylene, polypropylene or polyvinyl alcohol. Additionally, the outer sleeve 52 can be coated with a hydrophilic, lubricious coating such as HYDROPASS™ hydrophilic coating available from Boston Scientific Corporation, of Natick, Mass., and described in U.S. Pat. Nos. 5,702,754 and 6,048,620.

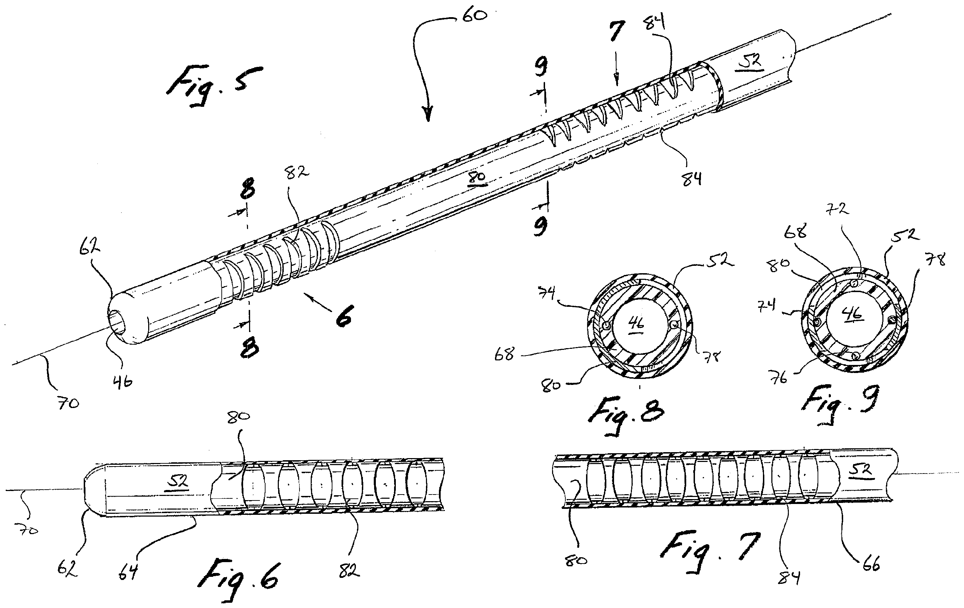

Referring now to FIGS. 5-9, the distal portion 60 includes a first active deflection section 64 and a second active deflection section 66. The first active deflection section 64 is capable of deflection in one plane relative to the handle 20 and the second active deflection section 66 is capable of deflection in the same plane or a different plane relative to the first active deflection section 64. As shown, the two planes are substantially perpendicular to each other, however, any degree of offset is acceptable depending on the desired application. In alternate embodiments, the first and/or second active deflection sections could each be more or less than two way deflectable.

The distal portion 60 of the flexible shaft 50 comprises an inner shaft 68 (FIGS. 8 and 9). When the first and second active deflection sections 64, 66 are oriented in the straight position as shown in FIG. 5, the inner shaft 68 defines a longitudinal axis 70. The inner shaft 68 includes a central lumen know as the working channel 46 and a plurality of smaller lumens 72, 74, 76, 78 extending longitudinally through the tubular wall of the inner shaft 68. As noted above, the proximal ends of pull wires 28, 30, 32, 34 are connected to activation hubs 24, 26 in the control system 22. The pull wires 28, 30, 32, 34 extend distally from the control system 22 and are each disposed in one of the smaller lumens 72, 74, 76, 78. The working channel 46 may have one or more lumens extending from the working port hub 44 to the distal end 62 and is used to insert ancillary products such as, for example, guide wires, graspers, cutters, irrigation, laser fibers and the like to facilitate a variety of diagnostic and therapeutic procedures. Illumination can also be achieved using fibers or electrical connection to an imaging sensor at the distal end 62. The inner shaft 68 is made from a biocompatible material acceptable for medical use with a low coefficient of friction such as polytetrafluoroethylene (PTFE) or polyethylene (PE). Other materials also may be appropriate.

In order to facilitate active deflection (i.e., steering) of the distal end 62, the distal portion 60 of the flexible shaft 50 includes an articulation layer 80 disposed over the inner shaft 68. The articulation layer has a first series of slots 82 in the articulation layer 80 located on opposing sides of the flexible shaft 50. The radial location of the slots 82 in the articulation layer 80 determine the direction of bending of the first active deflection section 64. In the embodiment shown in FIG. 5, the radial location of the slots 82 will allow the user to manipulate the first active deflection section 64 to the right and left.

The articulation layer 80 can be formed by various methods including extruding a cylinder with a central lumen in place and then cutting the cylinder tube with a knife, laser, milling tool, water jet, or other material removal mechanism to form the slots 82. Alternatively, the articulation layer 80 can be molded with the slots 82 in place. As will be appreciated, the shape, size, geometry (e.g., rounded or squared), and angle of the slots 82 may be uniform or may vary along the length of the articulation layer 80. Similarly, the distance between adjacent slots 82 may be uniform or may vary in order to tailor the bending and torque fidelity characteristics of the distal portion 60 of the flexible shaft 50. As with the inner shaft 68 discussed above, the articulation layer 80 should be made of a biocompatible material accepted for medical use that will bend but will not collapse. Suitable materials include polyurethane, polyethylene, polypropylene, or other biocompatible polymers. Other materials and/or fabrication techniques are possible.

In order to accomplish active deflection of the first active deflection section 64, pull wires 30, 34 disposed in smaller lumens 74, 78 respectively, extend from the first activation hub 24 along the length of the flexible shaft 50 and terminate at a location distal to the first active deflection section 64. As discussed above, when the user rotates the first activation hub 24 in a clockwise direction, tension is applied to pull wire 34, and tension is released from pull wire 30, thereby deflecting the first active deflection section 64 to the left. Conversely, when the user rotates the first activation hub 24 in the opposite, counter-clockwise direction, tension is applied to pull wire 30 and tension is released from pull wire 34, thereby deflecting the first active deflection section 64 to the right.

In order to facilitate additional active deflection (i.e., steering) of the distal portion 60 of the flexible shaft 50, the articulation layer 80 has a second series of slots 84 on opposing sides of the flexible shaft 50. To achieve bending in a second plane, the second series of slots 84 can be rotated relative to the first series of slots 82. In the embodiment shown in FIG. 5, the second series of slots 84 is rotated about 90 degrees relative to the first series of slots 82. In this orientation, the two planes of deflection will be substantially perpendicular to each other, therefore, the user will be able to manipulate the second active deflection section 66 in an upward and downward direction.

In order to accomplish active deflection of the second active deflection section 66, pull wires 28, 32 disposed in smaller lumens 72, 76 respectively, extend from the second activation hub 26 along the length of the flexible shaft 50 and terminate at a location distal to the second active deflection section 66, but proximal to the first active deflection section 64 (i.e., between the two active deflection sections). As discussed above, when the user rotates the second activation hub 26 in a clockwise direction, tension is applied to pull wire 28, and tension is released from pull wire 32, thereby deflecting the second active deflection section 66 in an upward direction. Conversely, when the user rotates the second activation hub 26 in the opposite, counter-clockwise direction, tension is applied to pull wire 32 and tension is released from pull wire 28, thereby deflecting the second active deflection section 66 in a downward direction.

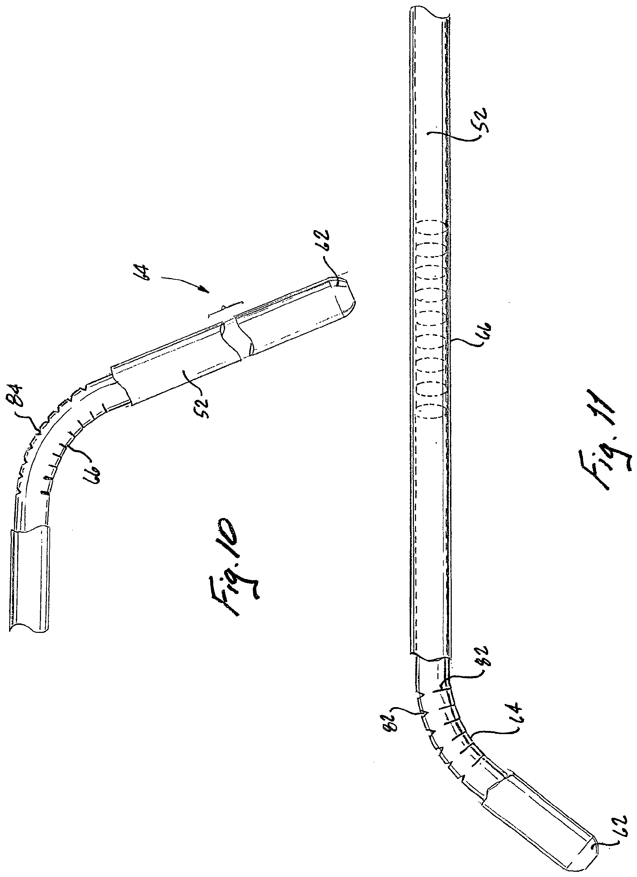

Referring now to FIG. 10, the second active deflection section 66 is shown bent in a downward direction and the first active deflection section 64 is in a straight position. To achieve this orientation, the user would rotate the second activation hub 26 in a counter-clockwise direction, thus applying tension to pull wire 32. The outer sleeve 52 is shown cut away in the region of the second active deflection section 66 showing that one side of the second series of slots 84 has been compressed by the tension applied to pull wire 32 and the opposing side of the second series of slots 84 has been expanded by the release of tension on pull wire 28. This type of bend is sometimes referred to as an "elbow" bend because of its location along the length of the flexible shaft 50.

Referring now to FIG. 11, the first active deflection section 64 is shown bent to the left and the second active deflection section 66 is in a straight position. To achieve this orientation, the user would rotate the first activation hub 24 in a clockwise direction, thus applying tension to pull wire 34. The outer sleeve 52 is shown cut away in the region of the first active deflection section 64 showing that one side of the first series of slots 82 has been compressed by the tension applied to pull wire 34 and the opposing side of the first series of slots 82 has been expanded by the release of tension on pull wire 32. This type of bend is sometimes referred to as an "wrist" bend because of its location along the length of the flexible shaft 50.

Prior to use, the tension of the pull wires 28, 30, 32, 34 is typically adjusted such that the first and second active deflection sections 64, 66 are both in substantially straight orientations relative to each other. This type of configuration is used to insert the distal end 62 of the endoscope 10 into the interior anatomy of a patient.

To ensure proper positioning, it is desirable for the endoscope 10 to be visible using fluoroscopy, echocardiography, intravascular ultrasound, angioscopy, or another means of visualization. Where fluoroscopy is utilized, any or all of the endoscope 10 may be produced with a material that is compounded with a radiopaque filler, or a radiopaque marker may be included on any portion of the device that would be useful to visualize. Examples of a radiopaque fillers that can be used are barium sulfate and bismuth subcarbonate. Radiopaque markers can be made from any of a number of materials including, for example, gold, platinum, or tungsten.

Referring now back to FIGS. 2A-2F, movements of the first and second active deflection sections 54, 56 will be described in greater detail. FIG. 2A shows the second active deflection section 66 bent in a downward direction and the first active deflection section 64 is in a straight position. FIG. 2B shows the first active deflection section 64 bent to the left and the second active deflection section 66 in a straight position.

FIGS. 2C-2F show more complex bending of the distal portion 60 in multiple planes of deflection. FIG. 2C shows the first active deflection section 64 bent to the left and the second active deflection section 66 bent downward. To achieve this orientation, the user would rotate the first activation hub 24 in a clockwise direction, thus applying tension to pull wire 34 and deflection the first active deflection section 64 to the left. The user would also rotate the second activation hub 26 in a counter-clockwise direction, thus applying tension to pull wire 32 and bending the second active deflection section 66 downward.

FIG. 2D shows the first active deflection section 64 bent to the right and the second active deflection section 66 bent upward. To achieve this orientation, the user would rotate the first activation hub 24 in a counter-clockwise direction, thus applying tension to pull wire 30 and bending the first active deflection section 64 to the right. The user would also rotate the second activation hub 26 in a clockwise direction, thus applying tension to pull wire 28 and bending the second active deflection section 66 upward.

FIG. 2E shows the first active deflection section 64 still bent to the right while the second active deflection section 66 is now bent downward. To achieve this orientation, the user would keep the first activation hub 24 rotated in a counter-clockwise direction as it was in reference to FIG. 2D, but the user would rotate the second activation hub 26 in a counter-clockwise direction. This counter-clockwise rotation would release the tension on pull wire 28 and apply tension to pull wire 32, thereby bending the second active deflection section 66 in a downward direction.

FIG. 2F shows the first active deflection section 64 bent to the left and the second active deflection section 66 bent upward. To achieve this orientation, the user would rotate the first activation hub 24 in a clockwise direction, thus applying tension to pull wire 34 and bending the first active deflection section 64 to the left. The user would also rotate the second activation hub 26 in a clockwise direction, thus applying tension to pull wire 28 and bending the second active deflection section 66 in an upward direction. As noted above, additional orientations and amount of bending of the first and second deflection sections 64, 66 are possible depending on the several variables including, for example, the amount of tension applied to the pull wires, the distance or spacing between the slots, axial location of the slots in the articulation layer 80, as well as the depth, width and shape of the slots. Furthermore, additional planes and/or locations or deflection along the length of the flexible shaft 50 can be achieved by increasing the number of pull wires and deflection sections.

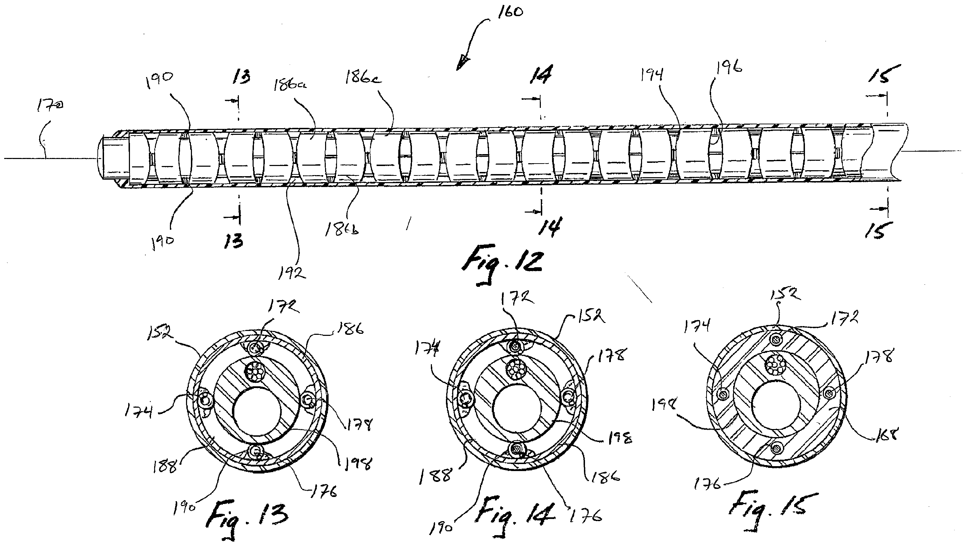

FIGS. 12-17 shows an alternative embodiment of a distal portion 160 of a flexible shaft 50 for use with an endoscope. The distal portion 160 is performs the same function as the distal portion 60 described above, and therefore like reference numerals preceded by the numeral "1" are used to indicate like elements. In this embodiment, the distal portion 160 is made of series of stacked rings, such as the articulation joints disclosed in U.S. patent application Ser. No. 10/956,011 (U.S. Patent Publication No. 2005-0131279.

In this embodiment, the distal portion 160 comprises a plurality of thin rigid rings 186a, 186b, 186c, etc., concentrically aligned defining an inner lumen 188. Each ring may be deep drawn, rolled and welded, or otherwise formed of stainless-steel or other biocompatible material that allows the ring to be rigid while having a thin wall profile in order to maximize the size of the inner lumen 188.

Each ring is connected to an adjacent ring with a pair of springs 190 laterally disposed on opposite sides of the inside wall of the rings. The springs 190 are welded, brazed, adhesively secured or otherwise bonded to an inner circumference of each ring segment joining adjacent rings together. The springs are secured at a predetermined radial location substantially aligned with the smaller lumens 172, 174, 176, 178 of the flexible shaft. For example, if three rings 186a, 186b, and 186c are aligned, the rings 186a and 186b are joined together with springs located at the 0 degree and 180 degree radial location on the rings, while ring 186b is joined to ring 186c with orthogonally aligned springs located at the 90 degree and 270 degree radial location on the rings. The springs are made of stainless steel or other biocompatible metal and springs of varying stiffness may be used along the length of the distal portion 160 to control the radius of curvature along the length of the distal portion.

A space is formed between adjacent rings so that the pair of springs 190 forms a flexible joint that can bend in directions that are away from the longitudinal axis 170 of the shaft 150 but has limited ability to compress the shaft 150 in the direction of the longitudinal axis 170 of the shaft 150.

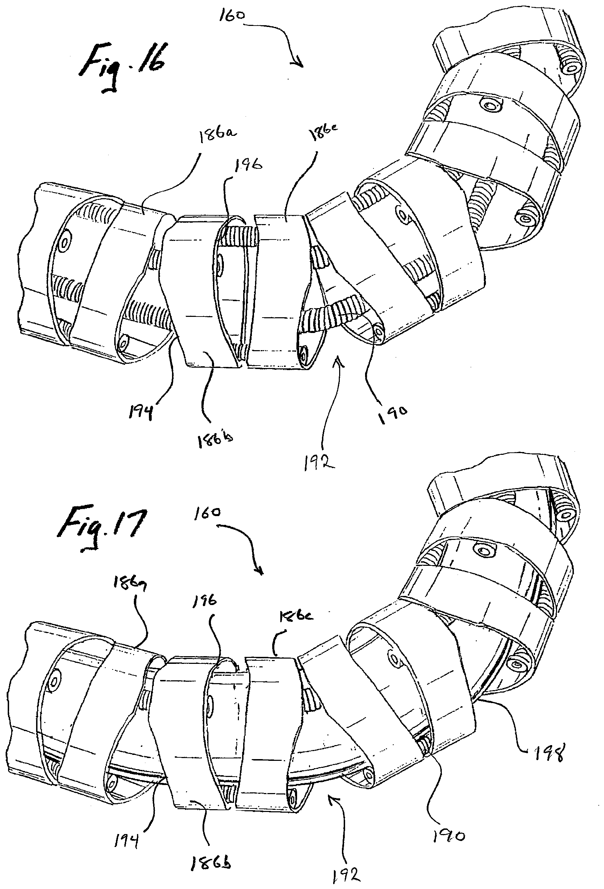

As shown in FIG. 12, when viewed from the side, each ring is not completely cylindrical but includes a front surface 194 and rear surface 196. Referring now also to FIGS. 16-17, the front 194 and rear 196 surfaces are sloped away from the point adjacent rings are joined by the springs, thereby forming a V-shaped gap 192 in which the distal portion 160 can bend. The sloped faces of the rings allow increased movement between adjacent rings and also provide a stop to prevent adjacent rings from sliding past each other.

Each spring 190 defines a small lumen with pull wires 128, 130, 132, 134 disposed therein. The distal portion of each pull wire is connected to the distal portion 160 of the flexible shaft section 150. As discussed above, in this embodiment, the two sets of pull wires (28, 32 and 30, 34) are rotated by 90 degrees allowing for two degrees of freedom (or deflection directions). In alternative embodiments, additional sets of pull wires and springs 190 may be included to allow for additional degrees of freedom.

A flexible outer sleeve 152 is disposed on the outside of the rings 186a, 186b, 186c, etc., to provide a smooth exterior surface. The outer sleeve 152 can be made from soft, thin polyurethane, LLDPE, silicon, pellethane, polyurethane or other approved biocompatible materials such as polyethylene, polypropylene or polyvinyl alcohol. Additionally, the outer sleeve 152 can be coated with a hydrophilic, lubricious coating such as HYDROPASS™ hydrophilic coating available from Boston Scientific Corporation, of Natick, Mass., and described in U.S. Pat. Nos. 5,702,754 and 6,048,620.

The flexible shaft 150 may further comprise an inner tube 198 running along the inside of the inner shaft 168 and the inner lumen 188. The inner tube has one or more lumens extending from the working port hub 144 to the distal end 162 and is used to insert ancillary products such as, for example, guide wires, graspers, cutters, irrigation, laser fibers and the like to facilitate a variety of diagnostic and therapeutic procedures. The inner tube 198 is made from a biocompatible material acceptable for medical use with a low coefficient of friction such as polytetrafluoroethylene (PTFE) or polyethylene (PE). Other materials also may be appropriate.

Active deflection of the distal portion 160 is accomplished in a similar manner as for distal portion 60 described above. Multiple active deflection sections (*i.e*., areas along the axis 170 where the distal portion 160 can bend in different planes or with different radius of curvature) can be achieved by the use of springs of varying tensions and by terminating the pull wires 128, 130, 132, 134 at different locations along the axis. For example, when pull wires 130, 134 disposed in smaller lumens 174, 178 respectively, extend from the first activation hub 124 along the length of the flexible shaft 150 and terminate at a location near the distal end 162 of the distal portion 160, a first active deflection section 164 is created. When pull wires 128, 132 disposed in smaller lumens 172, 176 respectively, extend from the second activation hub 126 along the length of the flexible shaft 150 and terminate at a location proximal to the first active deflection section 164, a second active deflection section 166 is created. As shown, these two active deflection sections 164, 166 are substantially perpendicular to each other and operate in the same manner as active deflection sections 64, 66 described above.

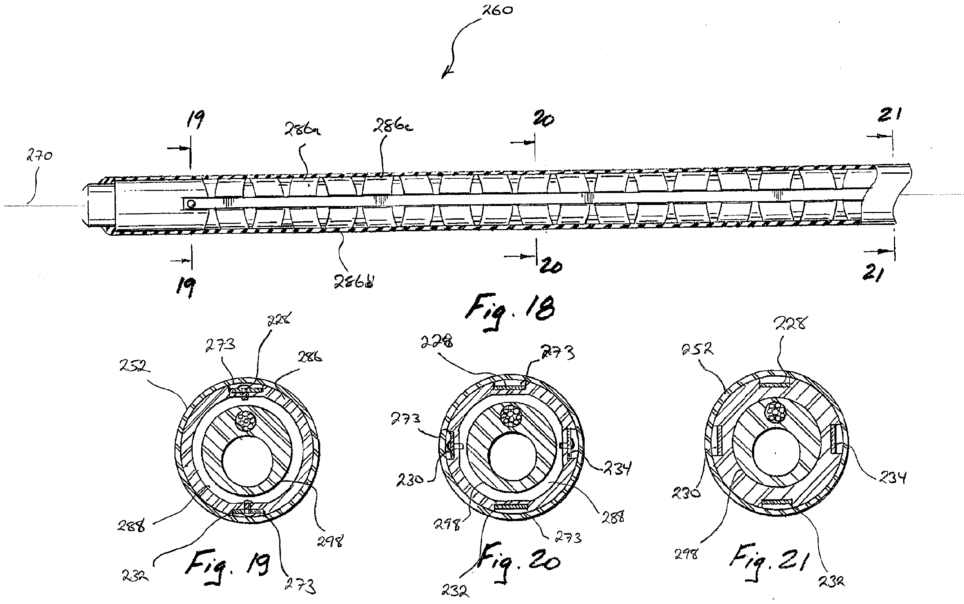

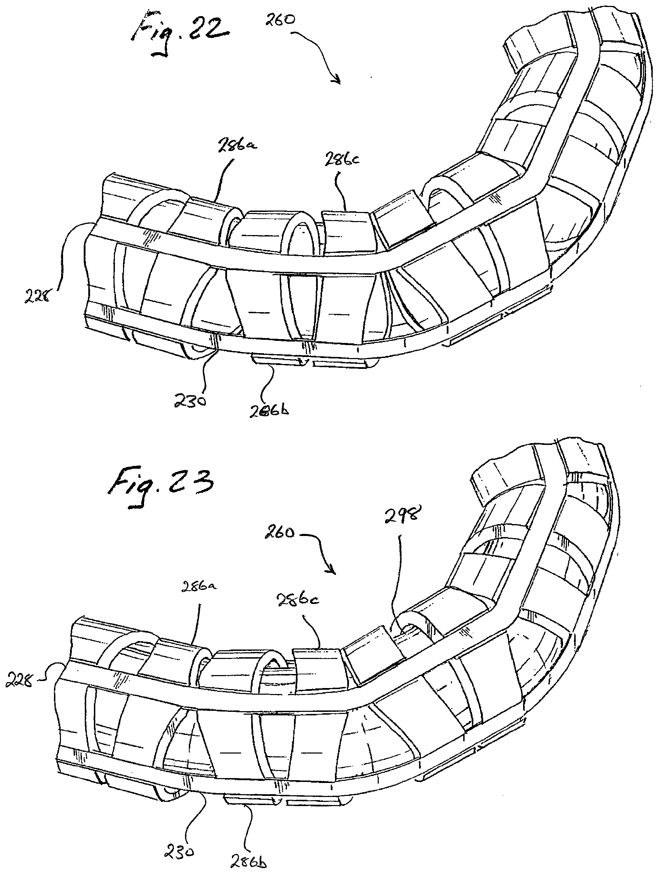

For smaller versions of a flexible shaft 150, the cross-section area occupied by the springs 190 and round pull wires 128, 130, 132, 134 may be prohibitive to other functional requirements of the device such as working channel, optics, etc. In these instances, an alternative embodiment that utilizes flat pull wires would be advantageous. FIGS. 18-23 shows an alternative embodiment of a distal portion 260 of a flexible shaft 150 for use with an endoscope 10 of the present invention. The distal portion 260 is performs the same function as the distal portion 160 described above, and therefore like reference numerals preceded by the numeral "2" are used to indicate like elements.

In this embodiment, the distal portion 260 is made of series of stacked rings 286a, 286b, 286c, etc. concentrically aligned defining an inner lumen 288. Each ring may be deep drawn, rolled and welded, or otherwise formed of stainless-steel or other biocompatible material that allows the ring to be rigid while having a thin wall profile in order to maximize the size of the inner lumen 288. Inwardly extending recesses 273 are positioned at predetermined intervals around the outer circumference of each of the rings 286 to receive flat pull wires 228, 230, 232, 234.

A flexible outer sleeve 252 is disposed on the outside of the rings 286a, 286b, 286c, etc., to provide a smooth exterior surface. The outer sleeve 252 can be made from soft, thin polyurethane, LLDPE, silicon, pellethane, polyurethane or other approved biocompatible materials such as polyethylene, polypropylene or polyvinyl alcohol. Additionally, the outer sleeve 252 can be coated with a hydrophilic, lubricious coating such as HYDROPASS™ hydrophilic coating available from Boston Scientific Corporation, of Natick, Mass., and described in U.S. Pat. Nos. 5,702,754 and 6,048,620.

In alternative embodiments, these flat pull wires 228, 230, 232, 234 could run completely along the inside of the rings or could weave from inside one ring to the outside of the next. In the embodiment where the flat pull wires 228, 230, 232, 234 run along the inside of the rings, the flexible shaft 250 may further comprise an inner tube 298 running along the inside of the rings 286a, 286b, 286c with groves to guide the location of the flat pull wires 228, 230, 232, 234. The inner tube 298 also has one or more lumens extending from the working port hub to the distal end 262 and is used to insert ancillary products such as, for example, guide wires, graspers, cutters, irrigation, laser fibers and the like to facilitate a variety of diagnostic and therapeutic procedures. The inner tube 298 is made from a biocompatible material acceptable for medical use with a low coefficient of friction such as polytetrafluoroethylene (PTFE) or polyethylene (PE). Other materials may be appropriate.

Active deflection of the distal portion 260 is accomplished in a similar manner as for distal portion 160 described above. Multiple active deflection sections (*i.e*., areas along the axis 270 where the distal portion 260 can bend in different planes or with different radius of curvature) can be achieved by the use of springs of varying tensions and by terminating the pull wires 228, 230, 232, 234 at different locations along the axis.

The disclosed embodiments are exemplary. The invention is not limited by or only to the disclosed exemplary embodiments. Also, various changes to and combinations of the disclosed exemplary embodiments are possible and within this disclosure.

## Claims

1. A flexible endoscope (10), comprising:
a handle (20); and
a flexible shaft (50) extending from the handle (20), the shaft (50) comprising a distal portion (60) having a tubular wall defining a central lumen and four smaller lumens (72, 74, 76, 78) extending longitudinally through at least a portion of the tubular wall, a pull wire (28, 30, 32, 34) disposed within each of the smaller lumens (72, 74, 76, 78), the distal portion (60) further comprising an articulation layer (80) formed by moulding or extruding a cylinder with a central lumen being disposed over the tubular wall, said articulation layer (80) including a first series of slots (82) located on opposite sides of the flexible shaft (50) which allow controlled bending of the distal portion (60) in a first active deflection section (64) of the distal portion (60) by movement of one or more of the pull wires (28, 30, 32, 34),
wherein the distal portion (60) further comprises in the articulation layer (80) a second series of slots (84) on opposing sides of the flexible shaft (50) which allow controlled bending of the distal portion (60) in a second active deflection section (66) of the distal portion (60),
wherein the second series of slots (84) is offset from the first series of slots by approximately 90° allowing controlled bending of the distal portion (60) in a first plane in the first active deflection section (64) and in a second plane in the second active deflection section (66), wherein a first pull wire (30) and a second pull wire (34) are configured to bend the flexible shaft (50) in the first plane and a third pull wire (28) and a fourth pull wire (32) are configured to bend the flexible shaft in the second plane.

2. The flexible endoscope (10) of claim 1, wherein the articulation layer (80) comprises a biocompatible polymer.

3. The flexible endoscope (10) of claim 1, wherein in order to accomplish active deflection of the first active deflection section (64), the first pull wire (30) and the second pull wire (34) terminate at a location distal to the first active deflection section (64), and wherein in order to accomplish active deflection of the second active deflection section (66), the third pull wire (28) and the fourth pull wire (32) terminate at a location distal to the second active deflection section (66), but proximal to the first active deflection section (64).

4. The flexible endoscope (10) of claim 1, wherein the first series of slots (82) comprises a first spacing between the slots and the slots having a first slot width, and the second series of slots (84) comprises a second different spacing and a second different slot width.

5. The flexible endoscope (10) of claim 1, wherein the first series of slots (82) or the second series of slots (84) are rounded or wherein the first series of slots (82) or the second series of slots (84) are squared.

6. The flexible endoscope (10) of claim 1, further comprising an outer sleeve (52) disposed on the outside of the flexible shaft (50) to provide a smooth exterior surface.

7. The flexible endoscope (10) of claim 6, wherein the outer sleeve (52) comprises a lubricated coating and/or wherein the outer sleeve comprises a drug coating.

8. The flexible endoscope (10) of claim 1, wherein the handle (20) further comprises a control system (22).

9. The flexible endoscope (10) of claim 1, further comprising radiopaque markers or further comprising a radiopaque material.

## Patentansprüche

1. Flexibles Endoskop (10), welches aufweist:
einen Handgriff (20); und
eine flexible Welle (50), die sich von dem Handgriff (20) weg erstreckt, wobei die Welle (50) einen distalen Bereich (60) mit einer rohrförmigen Wand, die ein mittleres Lumen und vier kleinere Lumen (72, 74, 76, 78), die sich in Längsrichtung durch zumindest einen Teil der rohrförmigen Wand erstrecken, wobei ein Zugdraht (28, 30, 32, 34) innerhalb jedes der kleineren Lumen (72, 74, 76, 78) angeordnet ist, aufweist, und der distale Bereich (60) weiterhin eine Gelenkschicht (80) aufweist, die durch Formen oder Extrudieren eines Zylinders mit einem mittleren Lumen, das über der rohrförmigen Wand angeordnet ist, gebildet ist, wobei die Gelenkschicht (80) eine erste Reihe von Schlitzen (82) enthält, die sich auf entgegengesetzten Seiten der flexiblen Welle (50) befinden und die ein gesteuertes Biegen des distalen Bereichs (60) in einem ersten aktiven Biegungsabschnitt (64) des distalen Bereichs (60) durch Bewegung eines oder mehrerer der Zugdrähte (28, 30, 32, 34) ermöglichen,
wobei der distale Bereich (60) weiterhin in der Gelenkschicht (80) eine zweite Reihe von Schlitzen (84) auf gegenüberliegenden Seiten der flexiblen Welle (50) aufweist, die ein gesteuertes Biegen des distalen Bereichs (60) in einem zweiten aktiven Biegungsabschnitt (66) des distalen Bereichs (60) ermöglichen,
wobei die zweite Reihe von Schlitzen (84) gegenüber der ersten Reihe von Schlitzen um ungefähr 90° versetzt ist, wodurch ein gesteuertes Biegen des distalen Bereichs (60) in einer ersten Ebene in dem ersten aktiven Biegungsabschnitt (64) und in einer zweiten Ebene in dem zweiten aktiven Biegungsabschnitt (66) ermöglicht wird, wobei ein erster Zugdraht (30) und ein zweiter Zugdraht (34) konfiguriert sind, die flexible Welle (50) in der ersten Ebene zu biegen, und ein dritter Zugdraht (28) und ein vierter Zugdraht (32) konfiguriert sind, die flexible Welle in der zweiten Ebene zu biegen.

2. Flexibles Endoskop (10) nach Anspruch 1, bei dem die Gelenkschicht (80) ein biokompatibles Polymer aufweist.

3. Flexibles Endoskop (10) nach Anspruch 1, bei dem, um eine aktive Biegung des ersten aktiven Biegungsabschnitts (64) zu erreichen, der erste Zugdraht (30) und der zweite Zugdraht (34) an einem Ort distal von dem ersten aktiven Biegungsabschnitt (64) enden, und bei dem, um eine aktive Biegung des zweiten aktiven Biegungsabschnitts (66) zu erreichen, der dritte Zugdraht (28) und der vierte Zugdraht (32) an einem Ort distal von dem zweiten aktiven Biegeabschnitt (66), aber proximal von dem ersten aktiven Biegeabschnitt (64) enden.

4. Flexibles Endoskop (10) nach Anspruch 1, bei dem die erste Reihe von Schlitzen (82) einen ersten Abstand zwischen den Schlitzen aufweist und die Schlitze eine erste Schlitzbreite haben, und die zweite Reihe von Schlitzen (84) einen zweiten, verschiedenen Abstand und eine zweite, verschiedene Schlitzbreite aufweist.

5. Flexibles Endoskop (10) nach Anspruch 1, bei dem die erste Reihe von Schlitzen (82) oder die zweite Reihe von Schlitzen (84) gerundet sind oder bei dem die erste Reihe von Schlitzen (82) oder die zweite Reihe von Schlitzen (84) quadratisch sind.

6. Flexibles Endoskop (10) nach Anspruch 1, weiterhin aufweisend eine äußere Manschette (52), die auf der Außenseite der flexiblen Welle (50) angeordnet ist, um eine glatte äußere Oberfläche zu erhalten.

7. Flexibles Endoskop (10) nach Anspruch 6, bei dem die äußere Manschette (52) eine Schmierbeschichtung aufweist und/oder bei dem die äußere Manschette eine Medikamentenbeschichtung aufweist.

8. Flexibles Endoskop (10) nach Anspruch 1, bei dem der Handgriff (20) weiterhin ein Steuersystem (22) aufweist.

9. Flexibles Endoskop (10) nach Anspruch 1, weiterhin aufweisend strahlenundurchlässige Markierungen oder weiterhin aufweisend ein strahlenundurchlässiges Material.

## Revendications

1. Endoscope flexible (10), comprenant :
un manche (20) ; et
un arbre flexible (50) qui s'étend depuis le manche (20), l'arbre (50) comprenant une partie distale (60) qui comporte une paroi tubulaire qui définit une lumière centrale et quatre lumières plus petites (72, 74, 76, 78) qui s'étendent longitudinalement au travers d'au moins une partie de la paroi tubulaire, un fil de traction (28, 30, 32, 34) qui est disposé à l'intérieur de chacune des lumières plus petites (72, 74, 76, 78), la partie distale (60) comprenant en outre une couche d'articulation (80) qui est formée en moulant ou en extrudant un cylindre avec une lumière centrale qui est disposée au-dessus de la paroi tubulaire, ladite couche d'articulation (80) incluant une première série de fentes (82) qui sont situées sur des côtés opposés de l'arbre flexible (50), ce qui permet une flexion contrôlée de la partie distale (60) dans une première section de fléchissement actif (64) de la partie distale (60) au moyen d'un déplacement d'un ou de plusieurs des fils de traction (28, 30, 32, 34), dans lequel :
la partie distale (60) comprend en outre, dans la couche d'articulation (80), une seconde série de fentes (84) sur des côtés opposés de l'arbre flexible (50), ce qui permet une flexion contrôlée de la partie distale (60) dans une seconde section de fléchissement actif (66) de la partie distale (60), dans lequel :
la seconde série de fentes (84) est décalée par rapport à la première série de fentes d'approximativement 90°, ce qui permet un fléchissement contrôlé de la partie distale (60) dans un premier plan dans la première section de fléchissement actif (64) et dans un second plan dans la seconde section de fléchissement actif (66), dans lequel un premier fil de traction (30) ainsi qu'un deuxième fil de traction (34) sont configurés de manière à fléchir l'arbre flexible (50) dans le premier plan et un troisième fil de traction (28) ainsi qu'un quatrième fil de traction (32) sont configurés de manière à fléchir l'arbre flexible (50) dans le second plan.

2. Endoscope flexible (10) selon la revendication 1, dans lequel la couche d'articulation (80) comprend un polymère biocompatible.

3. Endoscope flexible (10) selon la revendication 1, dans lequel, afin de réaliser un fléchissement actif de la première section de fléchissement actif (64), le premier fil de traction (30) et le deuxième fil de traction (34) se terminent en un emplacement qui est distal par rapport à la première section de fléchissement actif (64), et dans lequel, afin de réaliser un fléchissement actif de la seconde section de fléchissement actif (66), le troisième fil de traction (28) et le quatrième fil de traction (32) se terminent en un emplacement qui est distal par rapport à la seconde section de fléchissement actif (66) mais qui est proximal par rapport à la première section de fléchissement actif (64).

4. Endoscope flexible (10) selon la revendication 1, dans lequel la première série de fentes (82) comprend un premier espacement entre les fentes et les fentes présentent une première largeur de fente, et la seconde série de fentes (84) comprend un second espacement différent et les fentes présentent une seconde largeur de fente différente.

5. Endoscope flexible (10) selon la revendication 1, dans lequel la première série de fentes (82) ou la seconde série de fentes (84) est constituée par des fentes arrondies ou dans lequel la première série de fentes (82) ou la seconde série de fentes (84) est constituée par des fentes carrées.

6. Endoscope flexible (10) selon la revendication 1, comprenant en outre une gaine externe (52) qui est disposée sur l'extérieur de l'arbre flexible (50) de manière à assurer une surface extérieure lisse.

7. Endoscope flexible (10) selon la revendication 6, dans lequel la gaine externe (52) comprend un revêtement lubrifié et/ou dans lequel la gaine externe comprend un revêtement à base de médicament(s).

8. Endoscope flexible (10) selon la revendication 1, dans lequel le manche (20) comprend en outre un système de commande (22).

9. Endoscope flexible (10) selon la revendication 1, comprenant en outre des marqueurs radio-opaques ou comprenant en outre un matériau radio-opaque.
